# EUROPEAN PATENT SPECIFICATION

(11) **EP 1 124 836 B1**
(45) Date of publication and mention of the grant of the patent: **14.12.2005**
(21) Application number: 98955155.1
(22) Date of filing: 27.10.1998
(51) Int. Cl.: C07H 15/203, C07H 15/26, C07H 17/02, C07H 17/075, C12Q 1/34

(54) **CHROMOGENIC SUBSTRATES OF SIALIDASE AND METHODS OF MAKING AND USING THE SAME**
CHROMOGENE SIALIDASESUBSTRATE UND VERFAHREN ZU DEREN HERSTELLUNG UND VERWENDUNG
SUBSTRATS CHROMOGENES DE SIALIDASE ET LEURS PROCEDES DE PREPARATION ET D'UTILISATION

(43) Date of publication of application: 22.08.2001
(73) Proprietor: IBBEX, Inc. c/o Gryphus Diagnostics, L.L.C., Knoxville, TN 37919 (US); UAB Research Foundation, Birmingham, AL 35294-0111 (US)
(72) Inventor: JOHNSON, Stephen, C., Birmingham, AL 35216 (US); SAEED, Ashraf, Birmingham, AL 35209 (US); LUO, Ming, Birmingham, AL 35216 (US)
(74) Representative: Perry, Robert Edward
(86) International application number: PCT/US1998/022786
(87) International publication number: WO 2000/024753

(56) References cited:
- EP-A- 0 413 561
- WO-A-89/02473
- WO-A-92/12256
- GB-A- 2 008 103
- US-A- 4 351 823
- DATABASE WPI Week 9019 Derwent Publications Ltd., London, GB; AN 90-143137 XP002095923 & JP 02 088594 A (SNOW BRAND MILK PROD CO LTD), 28 March 1990
- PALESE, P. ET AL.: "Applications of a synthetic neuraminidase substrate" APPL. MICROBIOL., vol. 25, no. 2, 1973, pages 195-201, XP002095922

## Description

### Field of the Invention

This invention relates to compounds that are chromogenic substrates for sialidases of bacterial, viral, protozoan, and vertebrate (including human) origin, and to their use.

### Background of the Invention

Sialidase (EC, 3.2..1.18, also known as neuraminidase, acylneuraminyl hydrolase) is a protein enzyme produced by many organisms such as bacteria, viruses, protozoa, and vertebrates including humans (Hirst [1941] *Science* 94:22-23). This class of enzymes catalyzes the hydrolysis of a terminal sialic acid which is linked to oligosaccharides through an O-glycosidic bond. Crystal structure of sialidases shows that the enzyme has a highly conserved active site centered in a propeller like β-sheet twirl (Crennell *et al.* [1993] *Proc. Natl. Acad. Sci. USA* 90:9852-9856).

Sialidases perform many critical biological functions. In bacteria, sialidase helps bacterial adhesion to tissues, and provides additional nutritional sources (Crennell *et al.* [1994] *Structure* 2(6):535-544). In viruses, it helps the release of progeny viruses (Liu *et al.* [1995] *J. Virol.* 69:1099-1106). In practice, *Trypanosoma cruzi,* a sialidase (also known as trans-sialidase) removes sialic acid from infected cells and decorates its own surface with these sialic acids. In humans, sialidases are involved in protein digestion, immune responses, and cell proliferation. Abnormal production of sialidases may lead to serious human diseases such as sialidosis or increased *Pseudomonas aeruginosa* infection in cystic fibrosis patients.

Since sialidases are associated with many diseases, a color-producing substrate of sialidase would be an excellent diagnostic or prognostic reagent for sialidase-related diseases. For instance, sialidase level is elevated in bacterial vaginosis (Briselden *et al*. [1992] *J. Clin. Microbiol.* 30:663-666). Measurement of sialidase level in the vaginal samples could be used to diagnose bacterial vaginosis. In periodontal disease caused by bacterial infection, it has been shown that presence of sialidase increases the colonization of harmful bacteria (Liljemark *et al.* [1989] *Caries Res.* 23:141-145). The cell invasion form of *T. cruzi,* Trypomastigote, expresses high levels of trans-sialidase activity; therefore, measurement of trans-sialidase level could be used for diagnosis of *T. cruzi* infection and for monitoring disease progress (Cross & Takle [1993] *Ann. Rev. Microbiol.* 47:385-411). In cystic fibrosis patients, *Pseudomonas aeruginosa* infection is one of the leading causes of death. Sialidase was shown to be involved in the disease progress (Cacalano *et al*. [1992] *J. Clin. Invest.* 89:1866-1874). Sialidase is also related to the regulation of cell proliferation (Bratosin *et al.* [1995] *Glycoconj. J.* 12:258-267), the clearance of plasma proteins (Bonten *et al.* [1996] *Genes & Devel.* 10:3156-3169), and the catabolism of gangliosides and glycoprotein (Gornati *et al.* [1997] *Mol*. *Cell Biochem.* 166*:117-124).*

Currently, there is available a synthetic substrate of sialidase, 4-methylumbelliferyl-B-acetyl-neuraminicacid (4-MUN) (Lentz *et al*. [1987] *Biochemistry* 26:5351-5358), which produces a product with characteristic fluorescence spectrum upon hydrolysis. This change of fluorescence spectrum can only be measured with a specialized instrument (fluorospectrometer).

### Brief Summary of the Invention

According to the present invention, novel compounds (which can function as chromogenic sialidase substrates) are of the formula 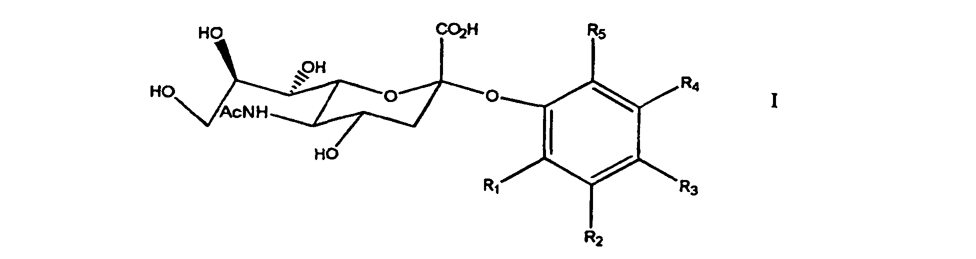 wherein at least two of R₁, R₂, R₄ and R₅ are H and the others are each R₆, OR₆, Cl, Br, I, F or NO₂;
R₃ is -CH=CHNO₂, 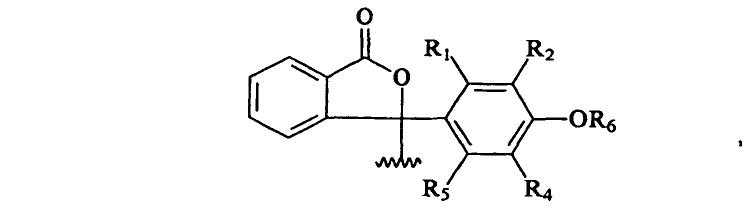 or 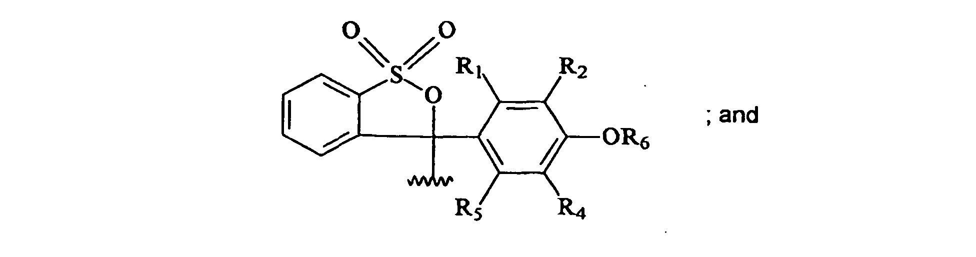 R₆ is H, C(CH₃)₃, CH(CH₃)₂, CH₂CH(CH₃)₂, CH(CH₃)(CH₂)₀₋₃CH₃ or (CH₂)₀₋₃CH₃; or a salt thereof.

A further aspect of the present invention is a compound of the invention and a carrier. Yet another aspect of the present invention is the use of a compound of the invention in the detection of a sialidase enzyme.

### Description of the Invention

Sialidases are produced by bacteria, viruses and protozoa; therefore, detecting the presence of sialidase in a biological sample can be indicative of the presence of these microbes. In specific embodiments, the detection of sialidases can be performed according to the subject invention in order to identify vaginal and periodontal infections, as well as to detect *Pseudomonas aeruginosa* in cystic fibrosis patients.

The presence of sialidase is detected, according to the invention, by the use of novel chromogenic substrate compounds. These compounds advantageously provide a visible color change when acted upon by sialidase. Thus, these substrates, when utilized according to the teachings of the subject invention, can be used to easily and accurately detect the presence of sialidase in a sample. In a preferred embodiment, the sample which is tested is a biological sample such as blood, mucus or saliva.

The compounds of the invention can be employed in a wide variety of assay formats. Typically, the assay will involve contacting a sample to be tested for the presence of sialidase with a chromogenic enzyme substrate of the subject invention. A color change occurring after the sample is contacted with the substrate is indicative of the presence of sialidase. The assay may optionally utilize positive and/or negative controls to aid in the interpretation and verification of the results. The results may also be quantified using standard optical measuring instrumentation.

General Methodologies. The following general methods are applicable to the synthesis of compounds of the invention. Modifications or variations of these methods can readily be utilized by those skilled in the art having the benefit of the instant disclosure.

Esterification. *N*-Acetyl-D-neuraminic acid is treated with methanol-washed Dowex 50W-X4 in methanol with stirring at room temperature for a period of time, generally 4 h. The mixture is filtered, and the filtrate is concentrated to give the desired esterified product after crystallization.

Those skilled in the art would recognize that other standard procedures are available for esterification of the same material, such as the use of other cation exchange resins, e.g., Amberlyst 15 or Dowex 50W-X8, among others.

*O*-Acetylation and Glycosyl Chloride Preparation. Treatment of the esterified product with acetyl chloride with stirring at room temperature under anhydrous conditions for a period of time, generally 20-24 h, results in formation of the per-*O*-acetylated glycosyl chloride. Note that in some instances the bubbling of dry hydrogen chloride (gas) into the reaction vessel is necessary to effect glycosyl chloride formation. Concentration of the reaction mixture with the water bath temperature not exceeding 35°C, and drying the residue *in vacuo* provides the product as a foam sufficiently pure for subsequent reactions.

Those skilled in the art would recognize that other standard procedures are available for *O*-acetylation and glycosyl chloride preparation of the same material, including a previously reported two-step procedure (Kuhn, *et al.*, 1966) which involves per-*O*-acetylation of the same material with acetic anhydride in perchloric acid, followed by formation of the glycosyl chloride by treatment with acetyl chloride.

*O*-Glycosylation. Treatment of the substituted hydroxybenzaldehyde derivative with sodium hydride in tetrahydrofuran with stirring at room temperature for a period of time, generally 1-3 h, results in formation of the sodium salt. Subsequent treatment of the sodium salt with the glycosyl chloride (compound 3) with stirring, for a period of time, generally 12-60 h, at room temperature results in *O*-glycosylation. Concentration of the reaction mixture, treatment of the residue with ethyl acetate and water, separation and drying of the organic phase, concentration of the organic phase, and column chromatography of the crude material affords the desired *O*-glycoside.

Those skilled in the art would recognize that other standard procedures are available for *O*-glycosylation of the same materials, such as traditional Lewis Acid-mediated *O*-glycosylation methodologies (Okamoto and Goto, 1990), as well as the use of alternate salts of the substituted aromatic hydroxyl derivative, including tetrabutylammonium (Baggett and Marsden, 1982) or silver (Holmquist and Brossmer, 1972) salts, among others.

De-*O*-acetylation and De-esterification. The protected *O*-glycoside is taken up in aqueous sodium hydroxide and stirred at room temperature for a period of time, generally 1-4 h. The mixture is then adjusted to pH 3-5 with Dowex 50W-X4 (H+) resin. Filtration, followed by lyophilization of the filtrate affords the desired de-*O*-acetylated and de-esterified material.

Those skilled in the art would recognize that other standard procedures are available for the complete de-*O*-acetylation and de-esterification of the same material, including a two-step procedure which involves complete de-*O*-acetylation of the same material with sodium methoxide in methanol or with an appropriate ion exchange resin, e.g. Amberlite IRA-400 (OH-), followed by de-esterification using conditions of acid hydrolysis or base hydrolysis.

### Synthesis of Chromogenic Substrates of Sialidases

Compounds with General Structure I and their salts and derivatives, may be prepared using any of several methods known in the art for the synthesis of substituted sialic acid analogs containing analogous structures.

To illustrate, synthetic approaches for selected examples from General Structure I are summarized in the following reaction scheme and are representative of the types of procedures which can be employed.

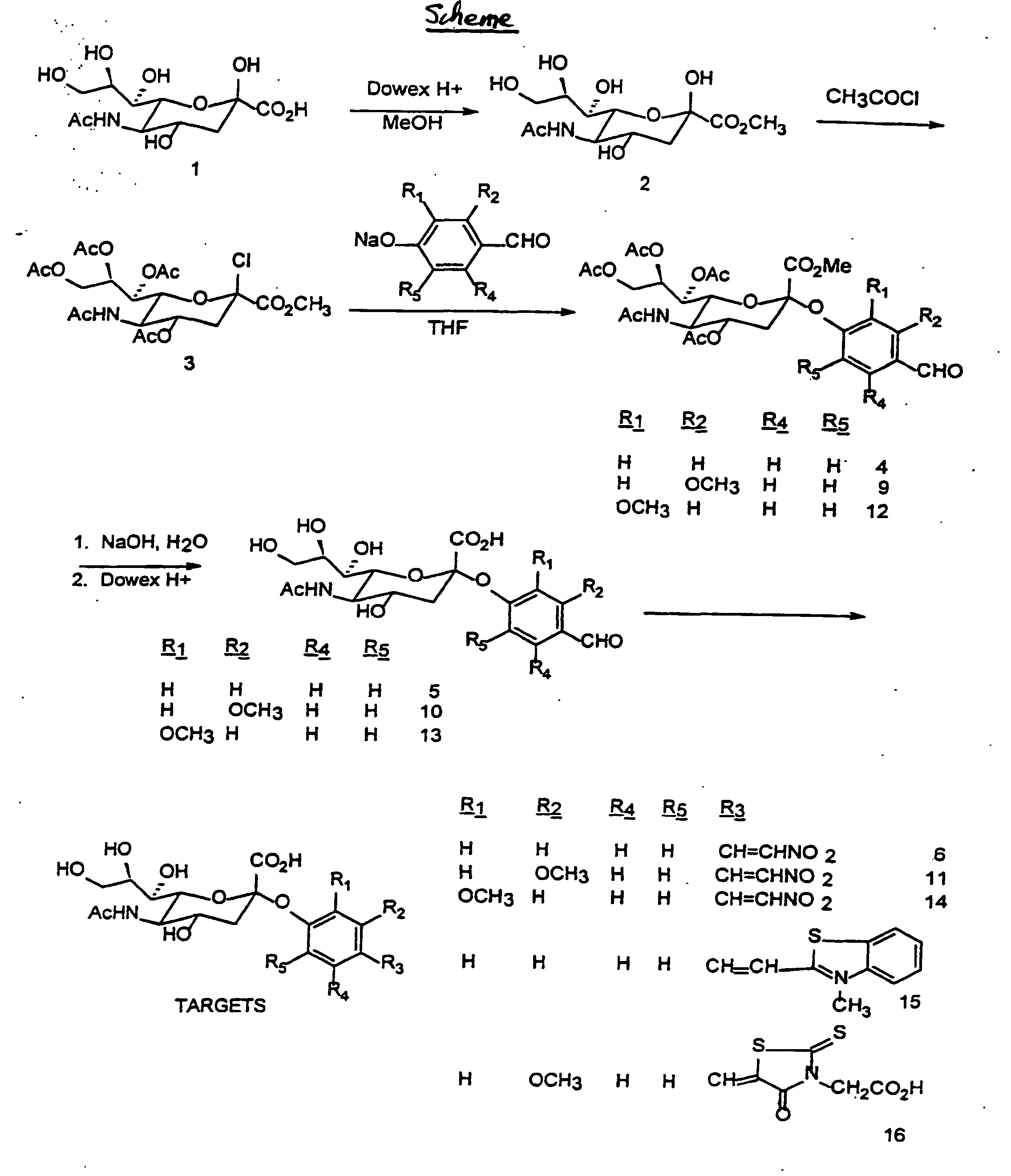

Compounds 6, 11 and 14 (shown in the reaction scheme) are specific examples of compounds of the invention. Further specific compounds of the invention have the following formulae 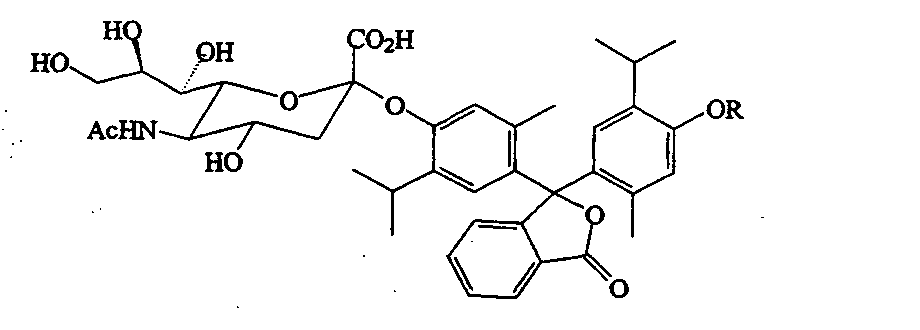 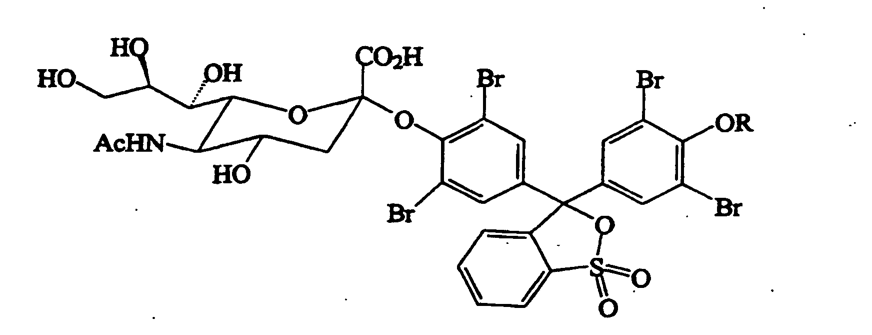 Advantageously, these compounds produce a blue color change when acted upon by a sialidase.

The reaction scheme illustrates constructing a basic skeleton of General Structure I via acid-mediated esterification of commercially available *N*-acetyl-D-neuraminic acid (1) to provide methyl *N*-acetyl-D-neuraminate (2), and subsequent per-*O*-acetylation and generation of the glycosyl chloride (3) according to modifications of known procedures (Kuhn *et al.,* 1966; Ogura *et al.,* 1986; Patel and Richardson, 1986). Treatment of compound 3 with the sodium salt of numerous substituted hydroxybenzaldehyde derivatives would provide the key intermediates to the desired targets (compounds 4, 9, and 12). Generation of the sodium salt would be accomplished with sodium hydride in tetrahydrofuran. This method of *O*-glycosylation has already been applied in the stereoselective preparation of numerous -*O*-glycosides of *N*-acetyl-D-neuraminic acid (Myers, *et al.,* 1980; Eschenfelder and Brossmer, *Carbohydr. Res.,* 1987; Eschenfelder and Brossmer, *Glycoconjugate J.,* 1987; Okamoto and Goto, 1990; Warner and O'Brien, 1979) derived from aromatic hydroxyls. However, none of the products described herein are contained in the aforementioned references. Synthetic approaches to or references to synthetic approaches to intermediates (I) and (2) are contained in the aforementioned references. Subsequent de-*O*-acetylation and de-esterification of the resulting intermediates can be accomplished with an aqueous sodium hydroxide solution and workup involving acidification of the reaction medium. This provides access to the formyl substituted phenolic-*O*-glycosides (compounds 5, 10, and 13).

Treatment of the derived targets (compounds 5, 10, and 13) with nitromethane, ammonium acetate, and acetic acid in ethanol under reflux provides access to the desired nitrovinyl targets (compounds 6,11, and 14). This procedure has been utilized in the preparation of nitrovinyl analogs of other monosaccharides (Patel and Richardson, 1986; Aamlid, *et al*., 1990) as chromogenic substrates for the assay of glycosidases; however, none of the products or intermediates described herein are contained in the aforementioned references.

It should also be noted that the *p*-nitrophenyl *O*-glycoside of *N*-acetylneuraminic acid (General Structure I, wherein, R₁ = R₂ = R₄ = R₅ = H and R₃ = NO₂ has been reported as a chromogenic substrate of sialidases (Eschenfelder and Brossmer, *Carbohydr. Res.,* 1987). Condensation of compounds 5,10, or 13 with any of numerous aromatic keto compounds in the presence of ammonia and ammonium chloride, provides ready access to numerous chromogenic substrates of sialidases (for representative examples, see compounds 15 and 16 Figure 2) of General Structure I.

Biochemical Evaluation for the Chromogenic Product of the Sialidase Substrate Compound. The source of sialidase was from purified recombinant bacterial sialidase from Salmonella T., whole influenza virus, or culture medium containing secreted human sialidase from 2CFSME0 cell line. The sialidase preparation was added to a buffer of 0.1 M sodium acetate at pH6.0, and the substrate compound 14 was provided at about 0.5 mM concentration. The reaction took place in room temperature for 20 mins in a volume of 100 µl. At the end of the reaction, the pH was adjusted by adding a solution ( 0.2 M glycine, and sodium hydroside with a pH value of 11.0). A color change to red was readily visible as examplified by Figures 1a and 1b. The color change was quantitated by measuring the light absorption of the reaction mixture. The light absorption was scanned with a photospectrometer. The peak value for compound IBX4010 is 495 nm. At a substrate concentration of 0.2 mM, the light absorption at 495 nm with a 1 cm path is 1.203. The control in which the reaction mixture was kept under the same condition for 10 minutes without addition of any enzyme had an absorption of 0.282 at 495 nm with a 1 cm path.

Compound 11 was tested by the same method. At the end of the reaction with pH adjusment, a color change to orange was readily visible as exemplified by Figures 1a and 1b. The color change was quantitated by measuring the light absorption of the reaction mixture. Ten minutes after the reaction, the mixture of the reaction product was adjusted to basic pH and the light absorption was scanned with a photospectrometer. The peak value for compound IBX4023 is 480 nm. At a substrate concentration of 0.2 mM, the light absorption at 480 nm with a 1 cm path is 4.065. The control in which the reaction mixture was kept under the same condition for 10 minutes without addition of any enzyme had an absorption of 1.452 at 480 mn with a 1 cm path.

Classes of Chromogenic Substrate Compounds of Sialidases. As used herein, the "effective amount" of a compound of the invention required for the use in the method presented herein will differ not only with the particular compound to be selected but also with the mode of application, and the nature of the sample specimen. The exact amount will be evaluated by testing with a sufficient number of clinical samples in each application as conducted by persons skilled in the art. However, a generally suitable concentration will range from about 0.1 to about 10 mM/ml of testing solutions. Furthermore, the compounds may be used as pure chemical applied to a test solution, or as a pure chemically acceptable salt or derivative. However, it is preferable to provide the active chemical or its chemically acceptable salt or derivative, as a medicinal formulation, either as a dry material (reaction solution provided seperately), or as a solution or suspension (an aqueous solution or other chemically acceptable solvent solutions), or as a dip stick. The subject specimen can be applied to the test for measuring the activity levels of sialidases. Those skilled in the art having the benefit of the instant disclosure will appreciate that amounts and modes of application are readily determinable without undue experimentation.

The following detailed examples for methods of preparation are for illustration only, and are not intended to represent a limitation of the invention. In all cases synthetic intermediates and products were found to be pure according to standards known to those skilled in the art (such as thin layer chromatography, melting or boiling points, gas chromatography, ion exchange chromatography, and/or high pressure liquid chromatography, elemental analysis, and spectroscopic methods). Furthermore, structures were characterized and assigned by spectroscopic methods considered standard practices by those skilled in the art (such as infrared, ultraviolet, and mass spectroscopies, ¹H and ¹³C nuclear magnetic resonance spectroscopy, and/or x-ray crystallography). Selected spectral data are described for intermediates and products.

### Example 1 - Preparations of methyl N-acetyl-β-D-neuraminate (2), methyl N-acetyl-4,7,8,9-tetra-O-acetyl-2-chloro-2-deoxy-neuraminate (3), methyl N-acetyl-4,7,8,9-tetra-O-acetyl-2-O-(4-formylphenyl)-α-D-neuraminate (4), and N-acetyl-2-O-(4-formylphenyl)-α-D-neuraminic acid (5)

Preparation of Methyl *N*-acetyl-β-D-neuraminate (2). To a stirred suspension of *N*-acetylneuraminic acid (1) (10.0 g, 32.3 mmol) in methanol (1.0 L) was added methanol-washed Dowex 50W-X4 (25.0 g) under a nitrogen atmosphere at room temperature protected from light. The resulting mixture was allowed to stir at room temperature for 4 h. The mixture was filtered and the filtrate was concentrated to dryness. The residue was crystallized from methanol to afford pure compound (2) (10.1 g, 96%): mp 178-180°C (d). A literature reference (Kuhn *et al.,* 1966) reports mp 179-180°C. A second literature reference (Ogura *et al*., 1986) reports mp 180-182°C.

¹H NMR (D₂O): (1.73 (dd, 1 H, *J*_{3a,4} 12.0 Hz, *J*_{3a,3c} 13.3 Hz, H-3a), 1.87 (s, 3 H, NAc), 2.14 (dd, 1 H, *J*_{3c,4} 5.0 Hz, H-3e), 3.33-3.48 (m, 2 H), 3.52-3.58 (m, 1H), 3.62-3.68 (m, I H), 3.65 (s, 3 H, CO₂CH)₃, 3.69-3.76 (m, 1H), 3.84-3.92 (m, 2 H).

Preparation of Methyl *N-*acetyl-4.7.8.9-tetra-*O*-acetyl-2-chloro-2-deoxy-D-neuraminate (3). A suspension of compound (2) (3.67 g, 11.3 mmol) in acetyl chloride (225 mL) was stirred under anhydrous conditions at room temperature protected from light for 24 h. The resulting solution was concentrated to dryness, the residue was coevaporated with anhydrous ether (2 X 50 mL), followed by coevaporations with anhydrous benzene (2 X 50 mL). Note that in all evaporations, the water bath temperature was maintained at or below 35 °C. The residue was dried in vacuo to afford pure compound (3) (4.8 g, 83%) as a syrup. A literature reference (Ogura *et al.,* 1986) reports mp 116-118°C; whereas, a second literature reference (Kuhn *et al.,* 1966) reports compound (3) as a syrup.

¹H NMR (CDCl₃): (1.92 (s, 3 H, NAc), 2.06, 2.07, 2.10, 2.14 (4 s, 12 H, 4 X OAc), 2.28 (dd, 1 H, *J*_{3a,4} 11.3 Hz, *J*_{3a-3c} 13.5 Hz, H-3a), 2.79 (dd, 1 H, *J*_{3c,4} 4.5 Hz, H-3e), 3.89 (s, 3 H, CO₂CH₃), 4.07 (dd, 1 H, *J*_{8.9}. 5.6 Hz, *J*_{9.9.}. 11.6 Hz, H-9'), 4.13-4.28 (m, 1 H, H-5), 4.36 (dd, 1 H, *J*_{6,7} 2.5 Hz, *J*_{5,6} 10.5 Hz, H-6), 4.43 (dd, 1 H, *J*_{8.9}. 2.9 Hz, H-9"), 5.18 (ddd, 1 H, *J*_{7,8} 6.7 Hz, H-8), 5.40 (ddd, 1 H, *J*_{4,5} 10.4 Hz, H-4), 5.49-5.52 (m, 2 H, H-7, NH).

Preparation of Methyl *N*-acetyl-4,7,8,9-tetra-*O*-acetyl-2-*O*-(4-formylphenyl)-α-D-neuraminate (4). To a stirred solution of 4-hydroxybenzaldehyde (121 mg, 0.98 mmol) in anhydrous tetrahydrofuran (6.0 mL) was added portionwise sodium hydride (48 mg of a 60% dispersion in mineral oil, 1.2 mmol) under a nitrogen atmosphere at room temperature. The resulting mixture was allowed to stir at room temperature for 25 min. The mixture was treated with compound (3) (500 mg, 0.98 mmol) and the resulting mixture was stirred under a nitrogen atmosphere atroom temperature for 52 h. The mixture was concentrated to dryness, the residue was diluted with ethyl acetate (15 mL), and washed with water (15 mL). The aqueous phase was extracted with ethyl acetate (3 X 15 mL), and combined organic phases were dried with magnesium sulfate, filtered, and the filtrate was concentrated to dryness. The residue was chromatographed (silica gel, 1:1 acetone-hexanes as eluting solvent) to afford pure compound (4) (238 mg, 41%): *R*_{*f*} *=* 026 (1:1 acetone-hexanes; UV, H₂SO₄).

¹H NMR (CDCl₃): ( 1.95 (s, 3 H, NAc), 2.07, 2.09, 2.13, 2.22 (4 s, 12 H, 4 X OAc), 232 (ut, I H, *J*_{3a,3c} =*J*_{3a,4} = 13.2 Hz, H-3a), 2.77 (dd, 1 H, *J*_{3a,4} 5.0 Hz, H-3e), 3.67 (s, 3 H, CO₂CH₃), 4.10-4.22 (m, 2 H), 4.24-4.32 (m, 1 H), 4.63 (dd, I H, *J* 2.0 Hz, *J* 11.7 Hz), 4.97-5.06 (m, 1 H), 5.30 (d, 1 H, *J* 12.6 Hz), 5.41 (s, 2 H), 7.20 (d, 2 H, *J* 9.6 Hz, 2 X ArH), 7.86 (d, 2 H, *J* 9.6 Hz, 2 X ArH), 9.95 (s, 1 H, CHO).

Preparation of *N*-Acetyl-2-O-(4-formylphenyl)-α-D-peuraminic acid (5). A solution of compound (4) (171 mg, 0.29 mmol) in aqueous sodium hydroxide (5.0 mL of a 1.0 M solution, 5.0 mmol) was stirred at room temperature for 2 h. The resulting mixture was cooled to 0°C and treated with methanol-washed Dowex 50W-X4 til pH 3. The mixture was filtered, the filtered resin was rinsed with water, and the filtrate was lyophilized to afford compound (5) (118 mg, 99%): *R*_{*f*} = 0.31 (5:2:1 ethyl acetate-methanol-0.02% aqueous calcium chloride; UV, H₂SO₄).

¹H NMR (D₂O): ( 2.08 (s, 3 H, NAc), 2.05-2.12 (m, 1 H, H-3a), 2.84 (dd, 1 H, *J*_{3c,4} 5.6 Hz, *J*_{3a,3c} 13.1 Hz, H-3e), 3.58-3.69 (m, 2 H), 3.82-3.90 (m, 3 H), 3.92-4.10 (m, 1 H), 4.21 (dd, 1 H, *J* 1.9 Hz, *J* 11.3 Hz), 7.32 (d, 2 H, *J* 9.6 Hz, 2 X ArH), 7.92 (d, 2 H, *J* 9.6 Hz, 2 X ArH), 9.84 (s, 1 H, CHO).

### Example 2 - Preparation of N-acetyl-2-O-[4-(2-nitrovinyl)phenyl]-α-D-neuraminic acid (6)

For the preparations of methyl *N*-acetyl-β-D-neuraminate (2), methyl *N*-acetyl-4,7,8,9-tetra-*O*-acetyl-2-chloro-2-deoxy-D-neuraminate (3), methyl N-acetyl-4,7,8,9-tetra-*O*-acetyl-2-*O*-(4-formylphenyl)-α-D-neuraminate (4), and *N*-acetyl-2-*O*-(4-formylphenyl)-α-D-neuraminic acid (5), see the experimental details for Example 1.

To a stirred solution of compound (5) (50 mg, 0.10 mmol) in a mixture of ethanol (2.0 mL) and acetic acid (0.05 mL) was added ammonium acetate (50 mg, 0.65 mmol) and nitromethane (0.20 mL, 3.70 mmol) at room temperature. The reaction mixture was heated under reflux for 30 min, cooled to room temperature, and evaporated to dryness. The residue was chromatographed (silica gel, 5:2:1 ethyl acetate-methanol-0.02% aqueous calcium chloride as eluting solvent) to afford pure compound (6) (32 mg, 68%): *R*_{*f*} = 0.50 (5:2:1 ethyl acetate-methanol-0.02% aqueous calcium chloride; UV, H₂SO₄).

¹H NMR (D₂O): ( 2.05 (s, 3 H, NAc), 1.95-2.02 (m, 1 H, H-3a), 2.89 (dd, 1 H, J_{3c,4} 5.2 Hz, J_{3a,3c} 12.9 Hz, H-3e), 3.57-3.69 (m, 2 H), 3.85-4.00 (m, 4 H), 4.06 (dd, 1 H, *J* 1.5 Hz, *J* 10.5 Hz), 7.22 (d, 2 H, *J* 9.0 Hz, 2 X ArH), 7.64 (d, 2 H, *J* 9.0 Hz, 2 X ArH), 7.83 (d, 1 H, *J* 13.5 Hz, H-vinylic), 8.13 (d, 1 H, *J* 13.5 Hz, H- vinylic).

### Example 3 - Preparation of 4-hydroxy-2-methoxybenzaldehyde (8), methyl N-acetyl-4,7,8,9-tetra-O-acetyl-2-O-(4-formyl-3-methoxyphenyl)-α-D-neuraminate (9), and N-acetyl-2-O-(4-formyl-3-methoxyphenyl)-α-D-neuraminic acid (10)

For the preparation of methyl *N*-acetyl-α-D-neuraminate (2), methyl *N*-acetyl-4,7,8,9-tetra-*O*-acetyl-2-chloro-2-deoxy-D-neuraminate (3), see the experimental details presented previously.

Preparation of 4-Hydroxy-2-methoxybenzaldehyde (8). To a stirred solution of 3-methoxyphenol (14.9 g, 120 mmol) in 15% aqueous potassium hydroxide (500 mL) was added chloroform (100 mL). The resulting solution was heated under reflux for 4 h, cooled to room temperature, and treated with 10% aqueous hydrochloric acid til pH 4. The suspension was filtered, and the filter cake was rinsed with chloroform (150 mL). The chloroform phase was separated, the aqueous phase was extracted with additional portions of chloroform (3 X 50 mL), and the combined organic phases were dried with magnesium sulfate. The solution was then filtered through a short column (silica gel, chloroform as eluting solvent) to afford compound (8). Crystallization from ethyl acetate gave pure compound (8) (1.8 g, 10%): mp 150-152°C. A literature reference (Patel and Richardson, 1986) reports mp 154-156°C. Additional references (Tiemann and Koppe, 1881; de Kiewiet and Stephen, 1931) report mp 153°C.

¹H NMR (CDCl₃): ( 3.87 (s, 3 H, OCH₃), 6.32-6.48 (m, 2 H, 2 X ArH), 7.62 (d, 1 H, J 9.6 Hz, ArH), 10.1 (s, 1 H, CHO).

Preparation of Methyl *N*-acetyl-4,7,8,9-tetra-*O*-acetyl-2-*O*-(4-formyl-3-methoxyphenyl)-α-D-neuraminate (9). To a stirred solution of compound (8) (900 mg, 5.92 mmol) in anhydrous tetrahydrofuran (35 mL) was added portionwise sodium hydride (288 mg of a 60% dispersion in mineral oil, 7.2 mmol) under a nitrogen atmosphere at room temperature. The resulting mixture was allowed to stir at room temperature for 2.5 h. The mixture was treated with compound (3) ( 2.33 g, 4.58 mmol) and the resulting mixture was stirred under a nitrogen atmosphere at room temperature for 115 h. The mixture was concentrated to dryness, the residue was diluted with ethyl acetate (40 mL), and washed with water (40 mL). The aqueous phase was extracted with ethyl acetate (3 X 40 mL), and combined organic phases were dried with magnesium sulfate, filtered, and the filtrate was concentrated to dryness. The residue was chromatographed (silica gel, 1:1 acetone-hexanes as eluting solvent) to afford pure compound (9) (1.23 g, 43%): *R*_{*f*} = 0.31 (1:1 acetone-hexanes; UV, H₂SO₄).

¹H NMR (CDCl₃): (1.96 (s, 3 H, NAc), 2.07, 2.09, 2.14, 2.18 (4 s, 12 H, 4 X OAc), 2.23-2.45 (m, 1 H, H-3a), 2.74 (dd, 1 H, *J*_{3c,4} 5.9 Hz, *J*_{3a,3c} 13.7 Hz, H-3e), 3.73 (s, 3 H, CO₂CH₃), 3.93 (s, 3 H, OCH₃), 4.12-4.22 (m, 2 H), 4.25-4.30 (m, 1 H), 4.60 (dd, 1 H, *J* 1.8 Hz, *J* 12.6 Hz), 4.96-5.08 (m, 1 H), 5.28-5.47 (m, 3 H), 6.65 (d, 1 H, *J* 3.0 Hz, ArH), 6.74 (dd, 1 H, *J* 3.0 Hz, *J* 9.6 Hz, ArH), 7.82 (d, 1 H, *J* 9.6 Hz, ArH), 10.33 (s, 1 H, CHO).

Preparation of *N*-Acetyl-2-O-(4-formyl-3-methoxyphenyl)-α-D-neuramic acid (10). A solution of compound (9) (751 mg, 1.20 mmol) in aqueous sodium hydroxide (20.0 mL of a 1.0 M solution, 20.0 mmol) was stirred at room temperature for 2 h. The resulting mixture was cooled to 0°C and treated with methanol-washed Dowex 50W-X4 til pH 3. The mixture was filtered, the filtered resin was rinsed with water, and the filtrate was lyophilized to afford compound (10) (288 mg, 54%): *R*_{*f*} = 0.34 (5:2:1 ethyl acetate-medianol-0.02% aqueous calcium chloride; UV, H₂SO₄).

¹H NMR (D₂O): ( 2.08 (s, 3 H, NAc), 2.04-2.12 (m, 1H, H-3a), 2.87 (dd, 1H, *J*_{3,4} 5.6 Hz, *J*_{3a,3e} 15.0 Hz, H-3e), 3.60-3.68 (m, 2 H), 3.81-3.95 (m, 4 H), 3.93 (s, 3 H, OCH₃), 4.23 (dd, 1H, *J* 3.7 Hz, *J* 11.2 Hz), 6.85 (dd, 1H, *J* 5.7 Hz, *J* 11.4 Hz, ArH), 6.97 (d, 1H, *J* 5.7 Hz, ArH), 7.75 (d, 1 H, *J* 11.4 Hz, ArH), 10.0 (s, 1 H, CHO).

### Example 4 - Preparation of N-acetyl-2-O-[3-methoxy-4-(2-nitrovinyl)phenyl]-α-D-neuraminic acid (11)

The overall reaction scheme is shown in Figure 9. For the preparation of methyl *N*-acetyl-β-D-neuraminate (2), methyl *N*-acetyl-4,7,8,9-tetra-*O*-acetyl-2-chloro-2-deoxy-D-neuraminate (3), see the experimental details presented previously.

For the preparations of 4-hydroxy-2-methoxybenzaldehyde (8), methyl *N*-acetyl-4,7,8,9-tetra-*O*-acetyl-2-*O*-(4-formyl-3-methoxyphenyl)-α-D-neuraminate (9), and *N*-acetyl-2-*O*-(4-formyl-3-methoxyphenyl)-α-D-neuraminic acid (10), see the experimental details presented in Example 3.

To a stirred solution of compound (10) (120 mg, 0.27 mmol) in a mixture of ethanol (4.8 mL) and acetic acid (0.12 mL) was added ammonium acetate (120 mg, 1.56 mmol) and nitromethane (0.48 mL, 8.86 mmol) at room temperature. The reaction mixture was heated under reflux for 30 min, cooled to room temperature, and evaporated to dryness. The residue was chromatographed (silica gel, 5:2:1 ethyl acetate-methanol-0.02% aqueous calcium chloride as eluting solvent) to afford pure compound (11) (48 mg, 36%): *R*_{*f*} = 0.64 (5:2:1 ethyl acetate-methanol-0.02% aqueous calcium chloride; UV, H₂SO₄).

¹H NMR (D₂O): ( 2.06 (s, 3 H, NAc), 1.98-2.03 (m, 1 H, H-3a), 2.88 (dd, 1 H, *J*_{3e,4} 5.1 Hz, *J*_{3a,3e} 14.0 Hz, H-3e), 3.59-3.68 (m, 2 H), 3.75-4.00 (m, 4 H), 3.95 (s, 3 H, OCH₃), 4.11 (dd, 1 H, *J* 2.5 Hz. *J* 11.4 Hz), 6.82 (d, 1 H, *J* 10.8 Hz, ArH), 6.95 (brs, 1 H, ArH), 7.54 (d, 1 H, *J* 10.8 Hz, ArH), 8.00 (d, 1 H, *J* 13.5 Hz, H-vinylic), 8.22 (d, 1 H, *J* 13.5 Hz, H-vinylic).

### Example 5 - Preparation of methyl N-acetyl-4,7,8,9-tetra-O-acetyl-2-O-(4-formyl-2-methoxyphenyl)-α-D-neuraminic acid (12) and N-acetyl-2-O-(4-formyl-2-methoxyphenyl α-D-neuraminic acid (13)

For the preparation of methyl *N*-acetyl-β-D-neuraminate (2), methyl *N*-acetyl-4,7,8,9-tetra-*O*-acetyl-2-chloro-2-deoxy-D-neuraminate (3), see the experimental details presented previously.

Preparation of Methyl *N*-acetyl-4,7,8-9-tetra-*O*-acetyl-2-*O*-(4-formyl-2-methoxyphenyl-α-D-neuraminic acid (12). To a stirred solution of vanillin (4-hydroxy-3-methoxybenzaldehyde) (273 mg, 1.8 mmol) in anhydrous tetrahydrofuran (12.0 mL) was added portionwise sodium hydride (86 mg of a 60% dispersion in mineral oil, 2.2 mmol) under a nitrogen atmosphere at room temperature. The resulting mixture was allowed to stir at room temperature for 2.5 h. The mixture was treated with compound (3) (700 mg, 1.38 mmol) and the resulting mixture was stirred under a nitrogen atmosphere at room temperature for 68 h. The mixture was concentrated to dryness, the residue was diluted with ethyl acetate (25 mL), and washed with water (25 mL). The aqueous phase was extracted with ethyl acetate (3 X 25 mL), and combined organic phases were dried with magnesium sulfate, filtered, and the filtrate was concentrated to dryness. The residue was chromatographed (silica gel, chloroform, followed by ethyl acetate as eluting solvent) to afford pure compound (12) (322 mg, 38%): *R*_{*f*} = 0.64 (1:8 acetone-ethyl acetate; UV, H₂SO₄).

¹H NMR (CDCl₃): ( 1.94 (s, 3 H, NAc), 2.08, 2.09, 2.14, 2.19 (4 s, 12 H, 4 X OAc), 2.33 (ut, I H, *J*_{3a,3c} = *J*_{3a,4} = 13.5 Hz, H-3a), 2.82 (dd, 1 H, *J*_{3c,4} 5.4 Hz, H-3e), 3.70 (s, 3 H, CO₂CH₃), 3.92 (s, 3 H, OCH₃), 4.10-4.18 (m, 2 H), 4.23-4.31 (m, 1 H), 4.52 (br d, 1 H, *J* 11.4 Hz), 4.97-5.12 (m, 1 H), 5.20-5.28 (m, 1 H), 5.30-5.40 (m, 2 H), 7.32 (d, 1 H, *J* 9.0 Hz, ArH), 7.41-7.48 (m, 2 H, 2 X ArH), 9.92 (s, 1 H, CHO).

Preparation of *N*-Acetyl-2-*O*-(4-formyl-2-methoxyphenyl)-α-D-neuraminic acid (13). A solution of compound (12) (236 mg, 0.38 mmol) in aqueous sodium hydroxide (6.0 mL of a 1.0 M solution, 6.0 mmol) was stirred at room temperature for 160 min. The resulting mixture was cooled to 0°C and treated with methanol-washed Dowex 50W-X4 til pH 3. The mixture was filtered, the filtered resin was rinsed with water, and the filtrate was lyophilized to afford compound (13) (152 mg, 91%): *R*_{*f*} = 0.46 (5:2:1 ethyl acetate-methanol-0.02% aqueous calcium chloride; UV, H₂SO₄).

¹H NMR (D₂O): ( 2.08 (s, 3 H, NAc), 2.03-2.12 (m, 1 H, H-3a), 2.92 (dd, 1 H, *J*_{3c,4} 5.2 Hz, *J*_{3a,3c} 13.9 Hz, H-3e), 3.54-3.70 (m, 2 H), 3.80-4.20 (m, 5 H), 3.92 (s, 3 H, OCH₃), 7.48 (d, 1 H, *J* 9.6 Hz, ArH), 7.52-7.60 (m, 2 H, 2 X ArH), 9.82 (s, 1 H, CHO).

### Example 6 - Preparation of N-acetyl-2-O-[2-methoxy-4-(2-nitrovinyl)phenyl]-α-D-neuraminic acid (14)

For the preparation of methyl *N*-acetyl-β-D-neuraminate (2), methyl *N*-acetyl-4,7,8,9-tetra-*O*-acetyl-2-chloro-2-deoxy-D-neuraminate (3), see the experimental details presented previously.

For the preparation of methyl *N*-acetyl-4,7,8,9-tetra-O-acetyl-2-*O*-(4-formyl-2-methoxyphenyl)-α-D-neuraminic acid (12) and *N*-acetyl-2-*O*-(4-formyl-2-methoxyphenyl)-α-D-neuraminic acid (13), see the experimental details presented in Example 5.

Preparation of *N*-Acetyl-2-*O*-[2-methoxy-4-(2-nitrovinyl)phenyl]-α-D-neuraminic acid (14). To a stirred solution of compound (13) (25 mg, 0.06 mmol) in a mixture of ethanol (2.0 mL) and acetic acid (0.02 mL) was added ammonium acetate (24 mg, 0.32 mmol) and nitromethane (0.10 mL, 1.9 mmol) at room temperature. The reaction mixture was heated under reflux for 30 min, cooled to room temperature, and evaporated to dryness. The residue was chromatographed (silica gel, 5:2:1 ethyl acetate-methanol-0.02% aqueous calcium chloride as eluting solvent) to afford pure compound (14) (19 mg, 70%): *R*_{*f*} = 0.64 (5:2:1 ethyl acetate-methanol-0.02% aqueous calcium chloride; UV, H₂SO₄).

¹H NMR (D₂O): (2.07 (s, 3 H, NAc), 1.97-2.04 (m, 1 H, H-3a), 2.87 (dd, 1 H, *J*_{3c,4} 5.1 Hz, *J*_{3a,3e} 14.0 Hz, H-3e), 3.54-3.69 (m, 2 H), 3.81-4.05 (m, 5 H), 3.93 (s, 3 H, OCH₃), 7.45 (d, 1 H, *J* 9.6 Hz, ArH), 7.48-7.55 (m, 2 H, 2 X ArH), 8.02 (d, 1 H, *J* 13.3 Hz, H-vinylic), 8.20 (d, 1 H, *J* 13.3 Hz, H-vinylic).

## Claims

1. A compound of the formula 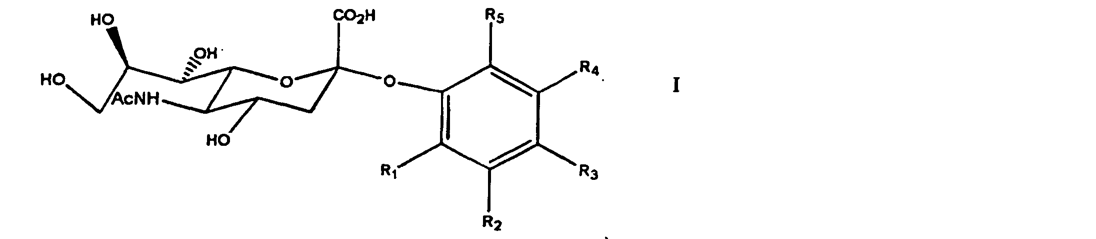 wherein at least two of R₁, R₂, R₄ and R₅ are H and the others are each R₆, OR₆, Cl, Br, I, F or NO₂;
R₃ is -CH=CHNO₂, 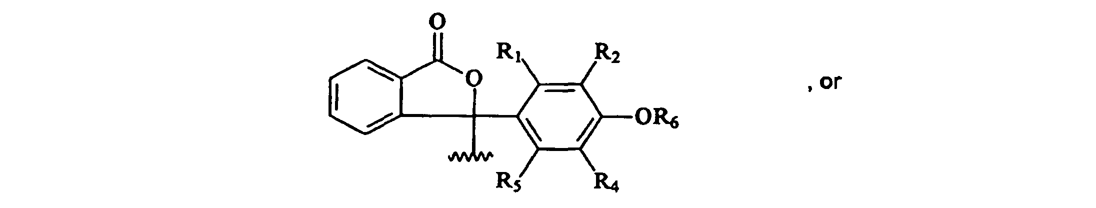 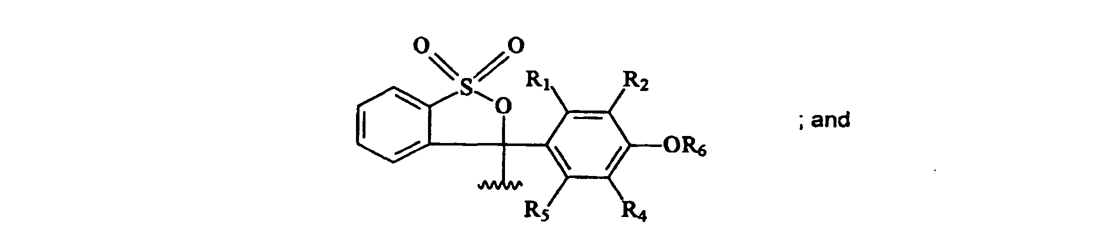 R₆ is H, C(CH₃)₃, CH(CH₃)₂, CH₂CH(CH₃)₂, CH(CH₃)(CH₂)₀₋₃CH₃ or (CH₂)₀₋₃CH₃;
or a salt thereof.

2. A compound according to claim 1, wherein R₁ and R₂ are H.

3. A compound according to claim 1, wherein R₁ and R₄ are H.

4. A compound according to claim 1, wherein R₁ and R₅ are H.

5. A compound according to claim 1, wherein R₂ and R₄ are H.

6. A compound according to claim 1, wherein R₂ and R₅ are H.

7. A compound according to claim 1, wherein R₄ and R₅ are H.

8. A compound according to claim 1, wherein R₁, R₂ and R₅ are H.

9. A compound according to claim 1, wherein R₁, R₄ and R₅ are H.

10. A compound according to claim 1, wherein R₂, R₄ and R₅ are H.

11. A compound according to claim 1, wherein R₁, R₂, R₄ and R₅ are H.

12. A compound according to any preceding claim, wherein R₃ is -CH=CHNO₂.

13. A compound according to any of claims 1 to 11, wherein R₃ is

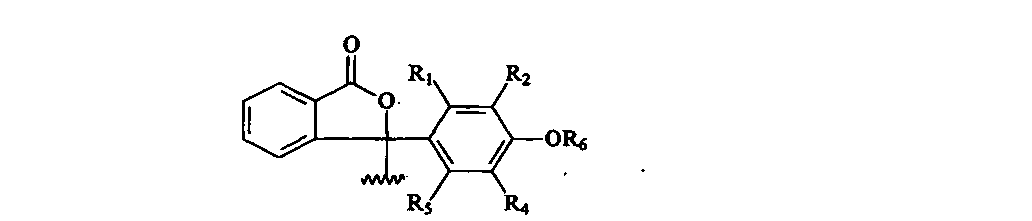

14. A compound according to any of claims 1 to 11, wherein R₃ is

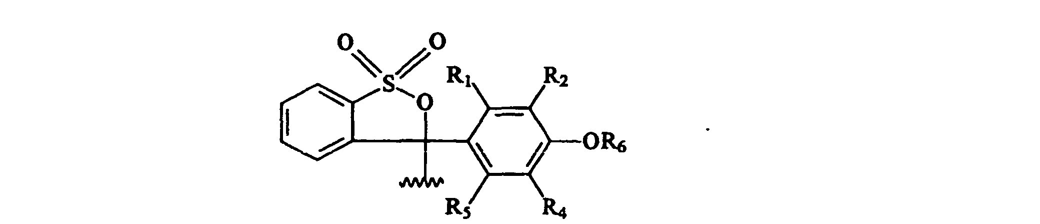

15. A compound according to claim 13 or claim 14, wherein R₆ is H.

16. A compound according to claim 13 or claim 14, wherein R₆ is -CH(CH₃)₂.

17. A compound according to claim 13 or claim 14, wherein R₆ is -(CH₂)₀₋₃CH₃.

18. A compound according to claim 17, wherein R₆ is CH₃.

19. A compound according to claim 17, wherein R₆ is C₂H₅.

20. A compound according to claim 17, wherein R₆ is C₃H₇.

21. A compound according to claim 17, wherein R₆ is C₄H₉.

22. A composition comprising a compound according to any preceding claim and a carrier.

23. The use of a compound according to any one of claims 1 to 21 in the detection of a sialidase enzyme.

## Patentansprüche

1. Verbindung der Formel 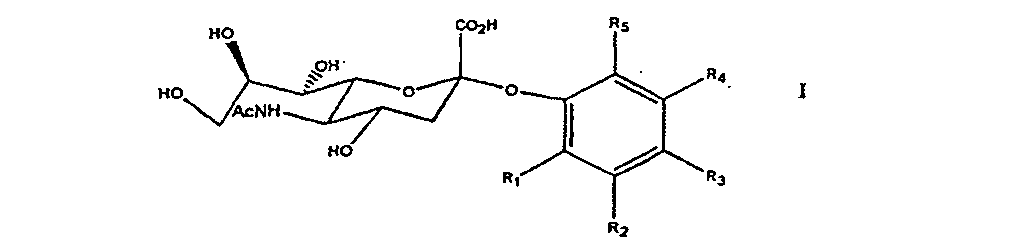 in der mindestens zwei von R₁, R₂, R₄ und R₅ für H stehen und die anderen jeweils R₆, OR₆, Cl, Br, I, F oder NO₂ sind;
R₃ für -CH=CHNO₂, 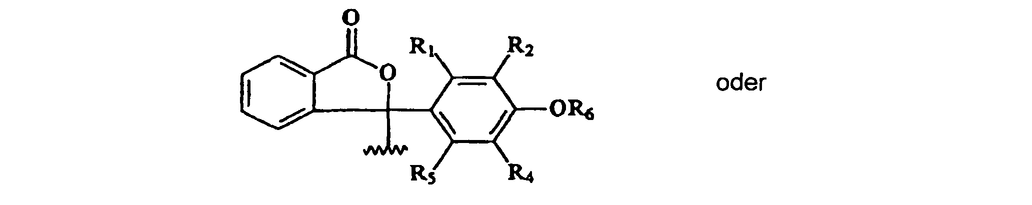 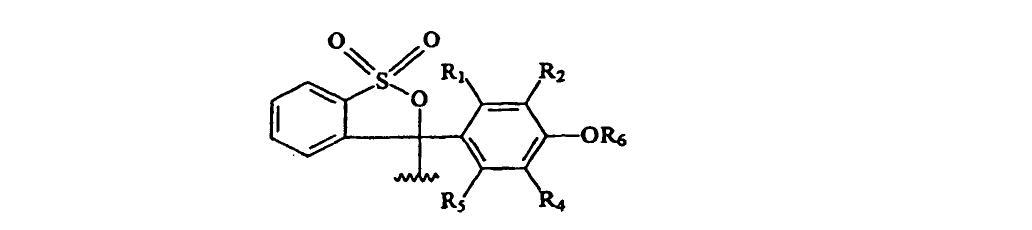 steht; und
R₆ für H, C(CH₃)₃, CH(CH₃)₂, CH₂CH(CH₃)₂, CH(CH₃)(CH₂)₀₋₃CH₃ oder (CH₂)₀₋₃CH₃ steht;
oder ein Salz derselben.

2. Verbindung nach Anspruch 1, in der R₁ und R₂ für H stehen.

3. Verbindung nach Anspruch 1, in der R₁ und R₄ für H stehen.

4. Verbindung nach Anspruch 1, in der R₁ und R₅ für H stehen.

5. Verbindung nach Anspruch 1, in der R₂ und R₄ für H stehen.

6. Verbindung nach Anspruch 1, in der R₂ und R₅ für H stehen.

7. Verbindung nach Anspruch 1, in der R₄ und R₅ für H stehen.

8. Verbindung nach Anspruch 1, in der R₁, R₂ und R₅ für H stehen.

9. Verbindung nach Anspruch 1, in der R₁, R₄ und R₅ für H stehen.

10. Verbindung nach Anspruch 1, in der R₂, R₄ und R₅ für H stehen.

11. Verbindung nach Anspruch 1, in der R₁, R₂, R₄ und R₅ für H stehen.

12. Verbindung nach irgendeinem vorangehenden Anspruch, in der R₃ für -CH=CHNO₂ steht.

13. Verbindung nach irgendeinem der Ansprüche 1 bis 11, in der R₃ für 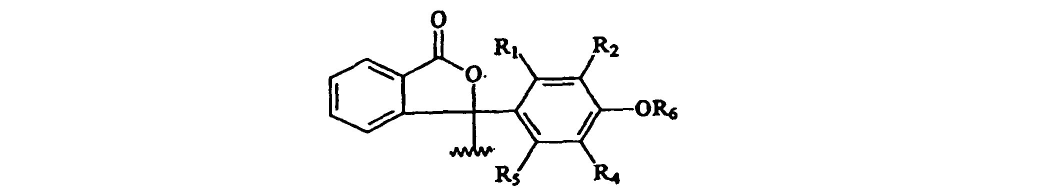 steht.

14. Verbindung nach irgendeinem der Ansprüche 1 bis 11, in der R₃ für 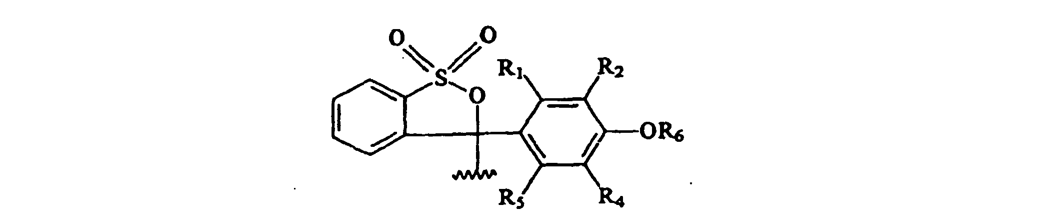 steht.

15. Verbindung nach Anspruch 13 oder Anspruch 14, in der R₆ für H steht.

16. Verbindung nach Anspruch 13 oder Anspruch 14, in der R₆ für -CH(CH₃)₂ steht.

17. Verbindung nach Anspruch 13 oder Anspruch 14, in der R₆ für -(CH₂)₀₋₃CH₃ steht.

18. Verbindung nach Anspruch 17, in der R₆ für CH₃ steht.

19. Verbindung nach Anspruch 17, in der R₆ für C₂H₅ steht.

20. Verbindung nach Anspruch 17, in der R₆ für C₃H₇ steht.

21. Verbindung nach Anspruch 17, in der R₆ für C₄H₉ steht.

22. Zusammensetzung, umfassend eine Verbindung nach irgendeinem vorangehenden Anspruch und einem Träger.

23. Verwendung einer Verbindung nach irgendeinem der Ansprüche 1 bis 21 beim Nachweis eines Sialidase-Enzyms.

## Revendications

1. Composé de formule 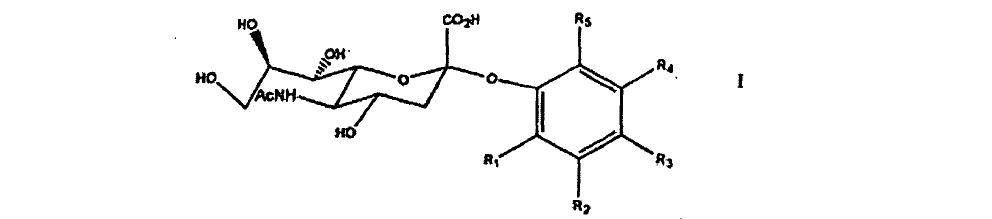 dans laquelle au moins deux de R₁, R₂, R₄ et R₅ représentent H et les autres représentent chacun un groupe R₆, OR₆, Cl, Br, I, F, ou NO₂ ;
R₃ représente un groupe -CH=CHNO₂, 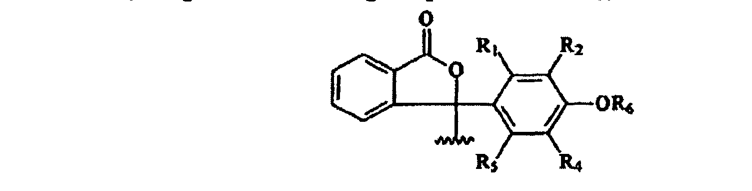 ou 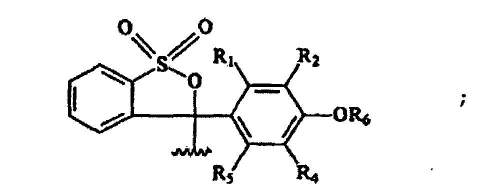 et
R₆ représente H, un groupe C (CH₃)₃, CH (CH₃)₂, CH₂CH (CH₃)₂, CH (CH₃) (CH₂)₀₋₃CH₃ ou ( CH_{2 0-3}CH₃ ;
ou un de ses sels.

2. Composé suivant la revendication 1, dans lequel R₁ et R₂ représentent H.

3. Composé suivant la revendication 1, dans lequel R₁ et R₄ représentent H.

4. Composé suivant la revendication 1, dans lequel R₁ et R₅ représentent H.

5. Composé suivant la revendication 1, dans lequel R₂ et R₄ représentent H.

6. Composé suivant la revendication 1, dans lequel R₂ et R₅ représentent H.

7. Composé suivant la revendication 1, dans lequel R₄ et R₅ représentent H.

8. Composé suivant la revendication 1, dans lequel R₁, R₂ et R₅ représentent H.

9. Composé suivant la revendication 1, dans lequel R₁, R₄ et R₅ représentent H.

10. Composé suivant la revendication 1, dans lequel R₂, R₄ et R₅ représentent H.

11. Composé suivant la revendication 1, dans lequel R₁, R₂, R₄ et R₅ représentent H.

12. Composé suivant l'une quelconque des revendications précédentes, dans lequel R₃ représente un groupe -CH=CHNO₂.

13. Composé suivant l'une quelconque des revendications 1 à 11, dans lequel R₃ représente un groupe

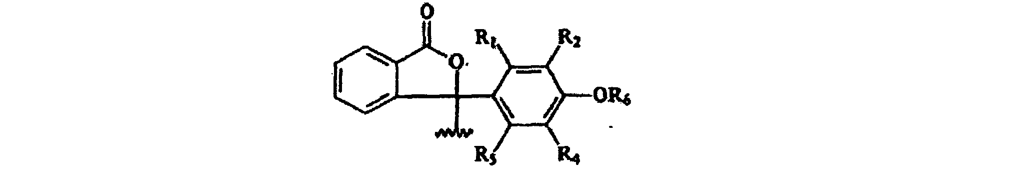

14. Composé suivant l'une quelconque des revendications 1 à 11, dans lequel R₃ représente un groupe

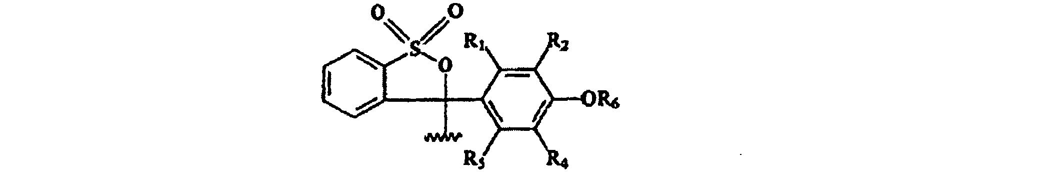

15. Composé suivant la revendication 13 ou la revendication 14, dans lequel R₆ représente H.

16. Composé suivant la revendication 13 ou la revendication 14, dans lequel R₆ représente un groupe -CH(CH₃)₂.

17. Composé suivant la revendication 13 ou la revendication 14, dans lequel R₆ représente un groupe (CH₂)₀₋₃CH₃.

18. Composé suivant la revendication 17, dans lequel R₆ représente un groupe CH₃.

19. Composé suivant la revendication 17, dans lequel R₆ représente un groupe C₂H₅.

20. Composé suivant la revendication 17, dans lequel R₆ représente un groupe C₃H₇.

21. Composé suivant la revendication 17, dans lequel R₆ représente un groupe C₄H₉.

22. Composition comprenant un composé suivant l'une quelconque des revendications précédentes et un support.

23. Utilisation d'un composé suivant l'une quelconque des revendications 1 à 21, dans la détection de l'enzyme sialidase.
